# EUROPEAN PATENT APPLICATION

(11) **EP 1 093 814 A1**
(43) Date of publication of application: **25.04.2001**
(21) Application number: 99121121.0
(22) Date of filing: 22.10.1999
(51) Int. Cl.: A61K 31/505, A61K 31/616, A61P 7/02

(54) **Use of dipyridamole or mopidamol in the manufacture of a medicament for the treatment and prevention of fibrin-dependent microcirculation disorders**

(71) Applicant: Boehringer Ingelheim Pharma KG, 55218 Ingelheim am Rhein (DE)
(72) Inventor: Eisert, Wolfgang, Prof.Dr.Dr.Dr., 30175 Hannover (DE)

(57) **Abstract**

A method of treatment of fibrin-dependent microcirculation disorders is disclosed, for example microcirculation disorders caused by metabolic diseases, inflammatory reactions or autoimmune diseases, which method comprises administering to a person in need of such treatment an effective amount of a pharmaceutical composition containing a pyrimido-pyrimidine selected from dipyridamole, mopidamol and the pharmaceutically acceptable salts thereof, and the use of said pyrimido-pyrimidine for the manufacture of a corresponding pharmaceutical composition.

## Description

### Field of the Invention

This invention relates to a method of treating fibrin-dependent microcirculation disorders using dipyridamole or mopidamol as active principle and the use of dipyridamole or mopidamol for the manufacture of a corresponding pharmaceutical composition.

### Background of the Invention

Dipyridamole {2,6-bis(diethanolamino)-4,8-dipiperidino-pyrimido[5,4-d]pyrimidine}, closely related substituted pyrimidopyrimidines and their preparation have been described in e.g. U.S.Patent 3,031,450. Further related substituted pyrimidopyrimidines and their preparation have been described in e.g. GB 1,051,218, inter alia the compound mopidamol {2,6-bis- (diethanolamino)-4-piperidinopyrimido[5,4-d]pyrimidine}. Dipyridamole was introduced as a *coronary vasodilator* in the early 1960s. It is also well known having *platelet aggregation inhibitor properties* due to the inhibition of adenosine uptake. Subsequently, dipyridamole was shown to reduce thrombus formation in a study of arterial circulation of the brain in a rabbit model. These investigations led to its use as an *anti-thrombotic agent;* it soon became the therapy of choice for such applications as stroke prevention, maintaining the patency of coronary bypass and valve-replacement, as well as for treatment prior to coronary angioplasty.

Furthermore, the European Stroke Prevention Study 2 (ESPS-2; J Neurol Sci. 1996; 143: 1-13; Neurology 1998; 51: 17-19) proved that treatment by dipyridamole alone was as effective as low-dose aspirin in the reduction of stroke risk, and combination therapy with dipyridamole and aspirin was more than twice as effective as aspirin alone.

Dipyridamole appears to inhibit thrombosis through multiple mechanisms. Early studies showed that it inhibits the uptake of adenosine, which was found to be a potent endogenous anti-thrombotic compound. Dipyridamole was also shown to inhibit cyclic AMP phosphodiesterase, thereby increasing intracellular c-AMP.

By laboratory models reflecting the complex physiology of the blood vessel it could be shown that the vasculature is not a passive conduit, but interacts profoundly with the blood through an intricate system of checks and balances to protect its integrity after vascular accident. Therefore the endothelium produces prostacyclin, a potent inhibitor of aggregation. The normal endothelium is not thrombogenic and prevents the attachment of platelets. Various stimulants precipitate the release of endothelium-derived relaxing factor (EDRF), which inhibits platelet adhesion and aggregation. At the same time, intracellular increase in cGMP was shown to be responsible for relaxation of smooth muscle cells following administration of nitro compounds. Thus the endothelium can inhibit thrombus formation by two separate mechanisms, one mediated by prostacyclin and c-AMP, and the other by EDRF and c-GMP. Dipyridamole appears to enhance both of these anti-thrombotic mechanisms of the vessel wall, in addition to its adenosine-sparing effects. It stimulates prostacyclin production by increasing intracellular levels of cAMP, and it enhances the strongly anti-thrombotic nitric oxide system by increasing cGMP.

Dipyridamole also has *antioxidant properties* (Free Radic. Biol. Med. 1995; 18: 239-247) that may contribute to its antithrombotic effect. When oxidized, low density lipoproteins become recognized by the scavenger receptor on macrophages, which is assumed to be the necessary step in the development of atherosclerosis (Ann. Rev. Med. 1992; 43: 219-25).

The inhibition of free radical formation by dipyridamole has been found to inhibit fibrinogenesis in experimental liver fibrosis (Hepatology 1996; 24: 855-864) and to suppress oxygen radicals and proteinuria in experimental animals with aminonucleoside nephropathy (Eur. J. Clin. Invest. 1998; 28: 877- 883; Renal Physiol. 1984; 7: 218-226). Inhibition of lipid peroxidation also has been observed in human nonneoplastic lung tissue (Gen. Pharmacol. 1996; 27: 855-859).

Mopidamol is known to possess antithrombotic and additionally antimetastatic properties.

### Summary of the Invention

It has now surprisingly been found that dipyridamole and mopidamol exert a protective effect on the vessel wall thereby strongly influencing the interaction of the vessel wall with the fibrin pathway of the coagulation system resulting in an essential reduction of fibrin accumulation after stimulating clot formation.

It is known that vascular damages accelerate fibrin accumulation since the prothrombinase complex becomes significantly more potent when settled on negatively charged phospholipids of the cellular membrane. By stabilizing the cellular membrane, less negatively charged phosphadidylserines may become exposed on the outer cell membrane, offering fewer opportunities for the prothrombinase complex to bind to the phospholipids, and thereby preventing the prothrombinase complex from operating at its full conversion rate to turn prothrombin into thrombin which is responsible for the conversion of fibrinogen into fibrin.

Platelet accretion and fibrin accumulation are the basic pathways involved in clot formation. It has been shown that the time course of these two pathways differs essentially (Thromb. Haemost. 1993; 69 (Abstr.): 569) proving that both mechanisms are not as stringently coupled as it was anticipated. Whereas the activity of dipyridamole and mopidamol as platelet aggregation inhibitor is well known it is a new finding that these agents additionally are *inhibitors of fibrin accumulation* mediated by their capacity to stabilize cellular membranes of the vessel wall. This effect is especially important in small vessels or capillary vessels where the ratio of vessel wall surface area to blood volume is high, and provides a new approach for treatment and prevention of fibrin-dependent microcirculation disorders. Therefore dipyridamole and mopidamol may have therapeutic potential in a variety of diseases involving progressive dysfunction of medium and small-sized vessels.

The finding that dipyridamole and mopidamol have significant inhibitory effects on fibrin accumulation via the vessel wall provides a rationale also for combination treatment together with other antithrombotic agents, such as platelet aggregation inhibitors, e.g. acetylsalicalic acid (ASA), clopidogrel or ticlopidine or the pharmaceutically acceptable salts thereof, fibrinogen receptor antagonists (Abciximab, RDGS-pepdides, synthetic i.v. or oral fibrinogen antagonists, e.g. fradafiban, lefradafiban or pharmaceutically acceptable salts thereof), heparin and heparinoids or antithrombins.

ASA inhibits aggregation through direct effects on the platelet, in more detail, by irreversibly acetylating platelet cyclooxygenase, thus inhibiting the production of thromboxane, which is strongly thrombotic. In high doses, however, aspirin crosses over into endothelial cells (N. Eng. J. Med. 1984; 311: 1206-1211), where it interrupts the production of prostacyclin, a potent natural inhibitor of platelet aggregation and by-product of the "arachidonic cascade" (N. Engl. J. Med. 1979; 300: 1142-1147). These observations led to the concept of low-dose antiplatelet therapy with ASA to maximize inhibition of thromboxane while minimizing the loss of prostacyclin (Lancet 1981; 1: 969-971). In combination with dipyridamole or mopidamol according to the invention also the low-dose ASA concept is preferred.

Viewed from one aspect the present invention provides a method of treatment of the human or non-human animal body, preferably mammalian body, for treating or preventing fibrin-dependent microcirculation disorders or of disease states where such microcirculation disorders are involved, said method comprising administering to said body an effective amount of a pharmaceutical composition comprising a pyrimido-pyrimidine selected from dipyridamole, mopidamol and the pharmaceutically acceptable sals thereof, dipyridamole being preferred, optionally in combination with one or more other antithrombotic agents.

Viewed from a different aspect the present invention provides the use of a pyrimido-pyrimidine selected from dipyridamole, mopidamol and the pharmaceutically acceptable salts thereof, dipyridamole being preferred, optionally in combination with one or more other antithrombotic agents, for the manufacture of a pharmaceutical composition for the treatment of the human or non-human animal body, preferably mammalian body, for treating or preventing fibrin-dependent microcirculation disorders or of disease states where such microcirculation disorders are involved.

### Detailed Description of the Invention

The invention provides a new approach for the treatment and prevention of fibrin-dependent microcirculation disorders associated with progressive dysfunction of medium and small-sized vessels comprising administering to a person in need of such treatment an effective amount of a pharmaceutical composition containing a pyrimido-pyrimidine selected from dipyridamole, mopidamol and the pharmaceutically acceptable salts thereof.

Fibrin-dependent microcirculation disorders are meant to be such disorders where fibrin deposition is involved in pathogenesis or progression of dysfunction of medium or small-sized vessels leading to a variety of clinical pictures. Metabolic diseases such as diabetes mellitus are known to cause said microcirculation disorders, however, also inflammatory reactions may cause microcirculation disorders due to local fibrinogen release from the tissue site of inflammation. Furthermore it is assumed that microcirculation disorders also can be caused by autoimmune reactions.

The indication "fibrin-dependent microcirculation disorders" should be understood in a non-limiting manner to comprise
microcirculation disorders caused by metabolic diseases where vascular damages are involved,
   such as diabetic angiopathy, especially diabetic microangiopathy, e.g. diabetic gangrene, diabetic retinopathy or ulcus cruris,
microcirculation disorders caused by inflammatory reactions,
   such as morbus crohn,
microcirculation disorders caused by autoimmune diseases,
   such as autoimmune chronic-active hepatitis (idiopathic hepatitis), primary-biliary cirrhosis or (autoimmune associated) multiple sclerosis,
peripheral microcirculation disorders,
   such as Raynaud's disease, tinnitus or
   sudden loss of hearing,
   or, as further indications,
   nephrosclerosis,
   prerenale hypertension,
   haemolytic-uremic syndrome (HUS),
   thrombotic-thrombocytopenic purpura (TTP) arterial hypertension,
   Sudeck's disease,
   central-veneous thrombosis of the eye and
   homocystine-induced vasculopathy.

As already mentioned hereinbefore dipyridamole, mopidamol or a pharmaceutically acceptable salt thereof can be used alone in a monopreparation or in combination with other antithrombotic agents for the treatment of fibrin-dependent microcirculation disorders.

It is of advantage to maintain a plasma level of dipyridamole or mopidamol of about 0.5 to 2 µmol/L, preferably of 0.8 to 1.5 µmol/L. This can be achieved using any of the oral dipyridamole retard, instant or the parenteral formulations on the market, the retard formulations being preferred, for instance those available under the trademark Persantin^{®}, or, for the combination therapy with ASA, using those formulations available under the trademark Asasantin^{®} or Aggrenox^{®}. Dipyridamol retard formulations are also disclosed in EP-A-0032562, instant formulations are disclosed in EP-A-0068191 and combinations of ASA with dipyridamole are disclosed in EP-A-0257344 which are incorporated by reference. In case of mopidamol also oral retard, instant or a parenteral formulations can be used, e.g. those disclosed in GB 1,051,218 or EP-A-0,108,898 which are incorporated by reference, retard formulations being preferred.

Dipyridamole or mopidamol can be administered orally in a daily dosage of 50 to 450 mg, preferably 50 to 240 mg, most preferred 75 to 200 mg. For long-time treatment it is of advantage to administer a dose of 25 mg three times a day. For parenteral administration dipyridamole could be given in a dosage of 1 to 5 mg/kg body weight, preferably 1 to 3.5 mg/kg body weight, during 24 hours as slow i.v. infusion (not faster than 0,2 mg/min).

Dipyridamole or mopidamol in combination with low-dose ASA may be administered orally in a daily dosage of 10 to 30 mg of ASA together with 50 to 300 mg of dipyridamole or mopidamol, preferably 80 to 240 mg of dipyridamole or mopidamol, for instance in a weight ratio between 1 to 5 and 1 to 12, most preferred a weight ratio of 1 to 8, for instance 25 mg of ASA together with 200 mg of dipyridamole or mopidamol.

Other antithrombotic compounds would be given at 0.1 to 10 times, preferably at 0.3 to 5.0 times, most preferred at 0.3 to 2.0 times the clinically described dose (e.g. Rote Liste^{®} 1999; fradafiban, lefradafiban: EP-A-0483667), together with a daily dosage of 50 to 450 mg, preferably 50 to 240 mg, most preferred 75 to 200 mg of dipyridamole or mopidamol.

In order to study the inhibition of fibrin accumulation by dipyridamole the following experiment was carried out:

### Study Using Radio Labeled Platelets and Fibrinogen In Vivo

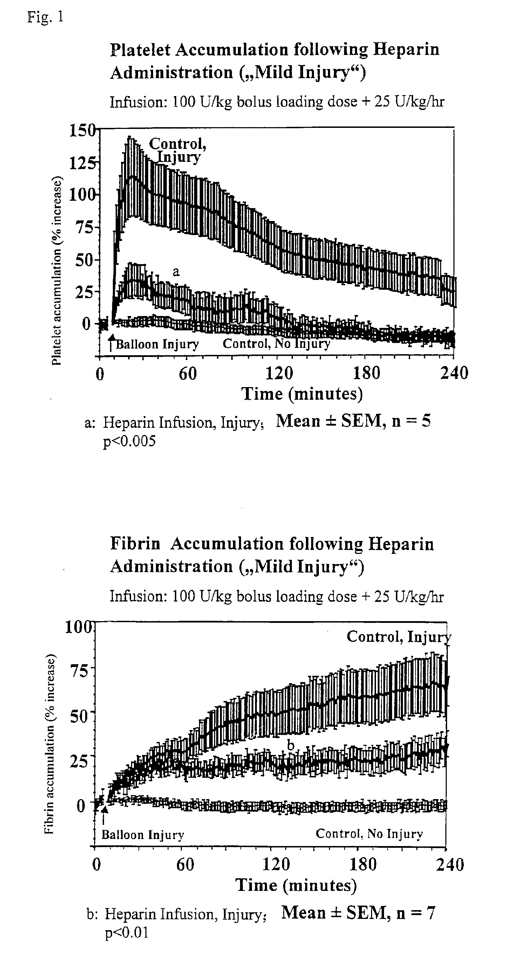

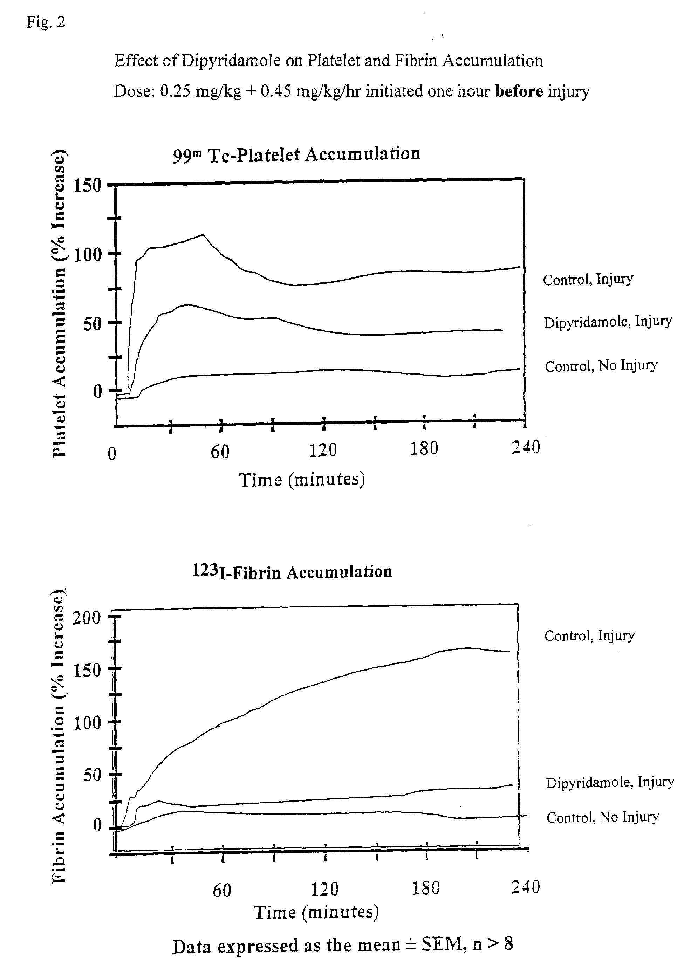

The effects of dipyridamole and heparin were investigated using platelets radiolabeled with ⁹⁹Tc and fibrinogen labeled with ¹²³J. The method is described in Nuclear Instruments and Methods in Physics Research A. 1994, 353: 448-452. An energy-sensitive solid-state radiation detector was placed to surround each carotid artery in rabbits. After inducing injury through balloon angioplasty, the accretion of platelets and fibrinogen was monitored for four hours. It was found that platelet and fibrinogen accretion had dissimilar time courses. Accretion was not detected in radiolabeled platelets injected 30 minutes after injury; apparently the angioplasty site had been passivated by endogenous platelet adherence. In contrast, fibrinogen accretion did not reach a plateau in control or treated animals even after four hours, indicating that different stimuli or triggers may be regulating fibrinogen at various time points after injury (Fig. 1). Treatment with heparin reduced accumulation of both platelets and fibrinogen. Treatment with dipyridamole also produced a reduction in platelet aggregation, which was similar to that seen with heparin; the reduction in accretion of fibrinogen was far greater with dipyridamole than with heparin, however (Fig. 2).

### FIGURE LEGENDS:

Fig. 1: Simultaneous detection of ⁹⁹Tc labeled platelets and ¹²³I-labeled fibrinogen at one minute interval after angioplasty of the common carotid artery of rabbits. Control (no treatment) group (N=6) showed after injury a rapid increase of platelets and a gradual build up of fibrinogen. Treatment with heparin (100U /kg bolus followed by 25U/ kg/h infusion) showed reduction of platelet as well as fibrinogen accretion. No injury measurements showing constant radioactivity.

Fig. 2: Deposition of radioactive labeled platelets (⁹⁹Tc) and fibrinogen (¹²³I) at angioplasty site after treatment with dipyridamole (0.25 mg/kg followed by 0.45 mg/kg/hr). Platelet deposition is reduced, but fibrinogen accretion is almost entirely blocked during the first four hours after angioplasty.

## Claims

1. A method of treatment of the human or non-human animal body for treating or preventing fibrin-dependent microcirculation disorders or of disease states where such microcirculation disorders are involved, said method comprising administering to said body an effective amount of a pharmaceutical composition comprising a pyrimido-pyrimidine selected from dipyridamole, mopidamol and the pharmaceutically acceptable salts thereof, optionally in combination with one or more other antithrombotic agents.

2. The method of claim 1, characterized in that the pyrimido-pyrimidine is dipyridamole.

3. The method of claim 1, characterized in that fibrin-dependent microcirculation disorder is selected from the group consisting of
microcirculation disorders caused by metabolic diseases where vascular damages are involved,
such as diabetic angiopathy, especially diabetic microangiopathy, e.g. diabetic gangrene, diabetic retinopathy or ulcus cruris,
microcirculation disorders caused by inflammatory reactions,
such as morbus crohn,
microcirculation disorders caused by autoimmune diseases,
such as autoimmune chronic-active hepatitis (idiopathic hepatitis), primary-biliary cirrhosis or (autoimmune associated) multiple sclerosis,
peripheral microcirculation disorders,
such as Raynaud's disease, tinnitus or
sudden loss of hearing, and, as further indications,
nephrosclerosis,
prerenale hypertension,
haemolytic-uremic syndrome (HUS),
thrombotic-thrombocytopenic purpura (TTP) arterial hypertension,
Sudeck's disease,
central-veneous thrombosis of the eye and
homocystine-induced vasculopathy.

4. The method of claim 1, characterized in that a plasma level of about 0.5 to 2 µmol/L of the pyrimido-pyrimidine is maintained.

5. The method of claim 1, characterized in that the pyrimido-pyrimidine is administered using an oral retard, instant or a parenteral formulation.

6. The method of claim 1, characterized in that the pyrimido-pyrimidine is administered orally in a daily dosage of 50 to 450 mg or parenterally in a dosage of 1 to 5 mg/kg body weight during 24 hours.

7. The method of claim 1, characterized in that the pharmaceutical composition comprises the pyrimido-pyrimidine in combination with acetylsalicylic acid (ASA) as the other antithrombotic agent, administered orally in a daily dosage of 10 to 30 mg of ASA together with 50 to 300 mg of the pyrimido-pyrimidine.

8. The use of a pyrimido-pyrimidine selected from dipyridamole, mopidamol and the pharmaceutically acceptable salts thereof, optionally in combination with one or more other antithrombotic agents, for the manufacture of a pharmaceutical composition for the treatment of the human or non-human animal body for treating or preventing fibrin-dependent microcirculation disorders or of disease states where such microcirculation disorders are involved.

9. The use of claim 8, characterized in that the pyrimido-pyrimidine is dipyridamole.

10. The use of claim 8, characterized in that fibrin-dependent microcirculation disorder is selected from the group consisting of
microcirculation disorders caused by metabolic diseases where vascular damages are involved,
such as diabetic angiopathy, especially diabetic microangiopathy, e.g. diabetic gangrene, diabetic retinopathy or ulcus cruris,
microcirculation disorders caused by inflammatory reactions,
such as morbus crohn,
microcirculation disorders caused by autoimmune diseases,
such as autoimmune chronic-active hepatitis (idiopathic hepatitis), primary-biliary cirrhosis or (autoimmune associated) multiple sclerosis,
peripheral microcirculation disorders,
such as Raynaud's disease, tinnitus or
sudden loss of hearing, and, as further indications,
nephrosclerosis,
prerenale hypertension,
haemolytic-uremic syndrome (HUS),
thrombotic-thrombocytopenic purpura (TTP) arterial hypertension,
Sudeck's disease,
central-veneous thrombosis of the eye and
homocystine-induced vasculopathy.
